(19) Europäisches Patentamt
European Patent Office
Office européen des brevets

(11) Publication number : 0 577 342 A1

# (12) EUROPEAN PATENT APPLICATION

(21) Application number : 93304958.7

(22) Date of filing : 24.06.93

(51) Int. Cl.[5] : C04B 35/00, A61L 27/00, A61L 25/00, A61K 6/033

(30) Priority : 29.06.92 GB 9213774

(43) Date of publication of application : 05.01.94 Bulletin 94/01

(84) Designated Contracting States : BE CH DE ES FR GB IT LI NL

(71) Applicant : QUEEN MARY AND WESTFIELD COLLEGE
Mile End Road
London E1 4NS (GB)

(72) Inventor : Bonfield, William, Prof.
48 Harmer Green Lane
Digswell, Welwyn, Herts. AL6 0AT (GB)
Inventor : Hastings, Garth Winton, Prof.
'Fairfields', Clayton Road
Newcastle upon Lyme, Staffs. ST5 4DH (GB)
Inventor : Knowles, Jonathan Campbell, Dr.
47 Primrose Road
South Woodford, London E18 1DD (GB)
Inventor : Santos, Jose Domingos da Silva
Rua D. Fernando 83, Leca do Balio
4465 S. Mamede De Infesta (PT)

(74) Representative : Allard, Susan Joyce et al
BOULT, WADE & TENNANT 27 Furnival Street
London EC4A 1PQ (GB)

## (54) Sintered hydroxyapatite compositions and method for the preparation thereof.

(57) A sintered enhanced hydroxyapatite which comprises hydroxyapatite, $Ca_{10}(P0_4)_6(OH)_2$, and a biocompatible glass based upon Ca0 and $P_20_5$.
The enhanced hydroxyapatite of the present invention may be used as bone implants, as bone fillers, as coatings for artificial joints or in pharmaceutical compositions for use as bone cements or dental cements.

EP 0 577 342 A1

The present invention relates to hydroxyapatite compositions and, in particular, to sintered composites of hydroxyapatite with a phosphate-based glass.

Hydroxyapatite is a member of the apatite group of minerals and has the chemical formula $Ca_{10}(P0_4)_6(OH)_2$. It is, essentially, a calcium phosphate including hydroxide having a Ca/P ratio of 1.67.

Hydroxyapatite is currently of much interest in the biomedical field due to its well established bioactivity. It has, for example, been used in various dental materials and in various medical applications, such as artificial joints and bones and as bone fillers. The material is, however, limited in its application because of its inadequate mechanical properties and thus has mainly been used for low load applications.

Attempts to enhance the mechanical properties of hydroxyapatite have generally been based upon the development of compaction methods and also the choice and control of grain size. Sintering aids have also previously been suggested for improving the mechanical properties of hydroxyapatite, but these have been single phase materials, such as CuO and $Al_20_3$.

We have now developed sintered hydroxyapatite compositions which are biocompatible and which have enhanced mechanical properties, and also a method for the preparation of these compositions.

Accordingly, the present invention provides a sintered enhanced hydroxyapatite which comprises hydroxyapatite, $Ca_{10}(P0_4)_6(OH)_2$, and a biocompatible glass based upon Ca0 and $P_20_5$.

In another aspect, the present invention provides a method for the preparation of an enhanced hydroxyapatite as defined above which comprises sintering a mixture of hydroxyapatite, $Ca_{10}(P0_4)_6(OH)_2$, and a biocompatible glass based upon Ca0 and $P_20_5$ at a temperature of above 1200°C.

In the preparation of the composite of the present invention, the biocompatible glass acts as a sintering aid and is generally contained in an amount of less than 10 percent by weight, preferably an amount of from 2.5 to 5% by weight, based on the weight of the hydroxyapatite.

By the term "biocompatible glass" as used herein is meant that the glass does not contain any metal ions which are not tolerated by the body (except for $Al^{3+}$, provided that the amount is small).

Suitable biocompatible glasses for use in the present invention are a two oxide $Ca0\text{-}P_20_5$ glass, a three oxide $Ca0\text{-}P_20_5\text{-}Na_20$ glass, or a four oxide $Ca0\text{-}P_20_5\text{-}Na_20\text{-}Al_20_3$ glass. Other components may be incorporated into the biocompatible glass based upon Ca0 and $P_20_5$, providing that the metals of the oxide used in the formation thereof are compatible with the body.

The preferred biocompatible glass for use in the present invention is a two oxide $CaO\text{-}P_2O_5$ glass. The mole ratio of CaO to $P_2O_5$ in these glasses may vary within wide limits but preferably is in the range of from 20:80 to 80:20 mol %. The glass having a mole ratio of $Ca0:P_20_5$ of 50:50 is particularly preferred.

The method for the preparation of the enhanced hydroxyapatites comprises sintering the aforesaid mixture of hydroxyapatite and the biocompatible glass based upon Ca0 and $P_20_5$ at a temperature of above 1200°C, preferably at a temperature in the range of from 1250° to 1350°C. Generally, the mixture to be sintered will be in a very fine particulate form with the particles having a particle size of less than 100 micrometres, preferably less than 75 micrometres, more preferably less than 10 micrometres.

The enhanced hydroxyapatites of the present invention have improved mechanical properties as compared to hydroxyapatite without the biocompatible glass addition.

The enhanced hydroxyapatites of the present invention may be used in any of the dental and medical applications for which unmodified hydroxy-apatite has previously been used. There may also be additional dental and medical fields in which the hydroxyapatite composites of the present invention may be used which are not open to the unmodified hydroxyapatite. Examples of fields where the enhanced hydroxyapatites of the present invention may be used are as bone implants, where an implant of the enhanced hydroxyapatite may be used, or as bone fillers where a powdered composition may be used as a filling material. The hydroxyapatite composite may also be used in the formation of artificial joints in which a coating of the hydroxyapatite composite is applied to at least a part of a metal or alloy joint.

The present invention also includes within its scope a composition which comprises an enhanced hydroxyapatite as hereinbefore defined together with a pharmaceutical acceptable diluent or carrier. Such compositions find use as bone cements or dental cements. Other applications of the enhanced hydroxyapatites of the present invention in the medical and dental field will be readily appreciated by those skilled in the art.

The present invention will be further described with reference to the following Examples.

## Examples 1 to 3

Phosphate based glasses were produced from reagent grade chemicals heated at 1300°C in an alumina crucible for 1 hour. The following compositions were prepared, in mole percent.

| | Example 1 | Example 2 | Example 3 |
|---|---|---|---|
| Ca0 | 54.5 | 28.0 | 10.1 |
| $P_20_5$ | 45.5 | 45.0 | 62.9 |
| $Na_20$ | - | 27.0 | 10.1 |
| $Al_20_3$ | - | - | 16.9 |

In each Example the glass was coarsely reduced to a sand type particle and milled in a porcelain ball mill pot for 24 hours. After milling its particle size was less than 100 micrometres and with median particle size, D0.5=6-9 micrometres according to Laser Diffraction analysis using a Malvern Mastersizer Ms-20. To hydroxyapatite powder each of the glass powders was added, in amounts of 2.5% and 5.0% by weight, with methanol (350 ml of methanol/200g of material) and the powders wet milled together for a further 24 hours. Following the milling process, the slip was poured and oven dried. The dried powder was then auto sieved to a particle size of less than 75 micrometres. Particle size distribution profiles were carried out after milling.

30mm diameter discs, using 4g of powder, were uniaxially pressed at 288 MPa and fired at 4°C/min ramp rate up to 1200° to 1350°C, with a one hour hold time at this temperature, followed by natural cooling inside the furnace.

Bending strength and Young's modulus measurements were obtained for each sample from a 4-point biaxial bending test in a concentric ring jig with a 20 mm supporting span and a 10 mm loading span at a cross head speed of 5 mm/min on 10 specimens. The concentric ring test eliminates many problems associated with the fracture induced by the edges of the samples although it gives lower results when compared with conventional bending tests. Fracture toughness measurements were performed using an indentation technique proposed by Laugier, J. Mater. Sci., 1987, 6, 355-356, using a 9.8 N load.

The materials were characterized by X-Ray Diffraction (XRD) and their microstructure analysed using SEM. In vitro tests were carried out in a phosphate buffered saline solution and samples were bending strength tested after 1 month.

The usual particle size distribution profile for the hydroxyapatite glass powders after being milled is shown in Figure 1 of the accompanying drawings.

SEM analysis showed that the hydroxyapatite/glass materials were very dense when sintered at 1250°C, or above. The flexural strength and Young's modulus for the compositions are given in Tables 1 and 2 below, respectively.

**Table 1**

Biaxial flexure for hydroxyapatite (HA) and hydroxyapatite/glass composites

Biaxial Flexure (MPa)

| | Material | 1200° | 1250° | 1300° | 1350° |
|---|---|---|---|---|---|
| 2.5% | HA | 24±4 | 24±5 | 28±7 | 28±7 |
| | 2-Oxide glass | 27±6 | 54±13 | 73±13 | 66±17 |
| | 3-Oxide glass | 36±10 | 38±7 | 55±18 | 49±14 |
| | 4-Oxide glass | 30±6 | 51±10 | 58±17 | 56±15 |
| 5% | 2-Oxide glass | 27±8 | 61±23 | 85±20 | 96±17 |
| | 3-Oxide glass | 29±8 | 54±16 | 69±21 | 61±18 |
| | 4-Oxide glass | 37±9 | 74±15 | 74±11 | 33±5 |

**Table 2**

Young's Modulus for hydroxyapatite (HA) and hydroxyapatite/glass composites

Young's Modulus (GPa)

| | Material | $1200^{\circ}$ | $1250^{\circ}$ | $1300^{\circ}$ | $1350^{\circ}$ |
|---|---|---|---|---|---|
| | HA | 59±7 | 87±10 | 88±8 | 89±9 |
| 2.5% | 2-Oxide glass | 66±7 | 117±15 | 121±15 | 122±10 |
| | 3-Oxide glass | 95±7 | 87±9 | 113±15 | 100±15 |
| | 4-Oxide glass | 79±8 | 103±14 | 105±11 | 108±12 |
| 5% | 2-Oxide glass | 75±14 | 120±12 | 120±14 | 134±9 |
| | 3-Oxide glass | 86±6 | 106±12 | 122±15 | 122±10 |
| | 4-Oxide glass | 83±12 | 113±13 | 110±11 | 75±10 |

A considerable improvement in the flexural strength was achieved by the incorporation of the glassy phase during the sintering of hydroxyapatite.

A slight increase in weight of the samples was detected after immersion in saline for 1 month, which was related to the precipitation of crystals on the surface of the materials. No changes in the flexural strength were found after 1 month.

All hydroxyapatite/glass materials showed a higher fracture toughness when compared with hydroxyapatite, as shown in Table 3.

**Table 3**

Klc for hydroxyapatite and hydroxyapatite/glass composites.

| | Material | Klc (MPa $m^{1/2}$) 1250° | 1300° | 1350° |
|---|---|---|---|---|
| | HA | 0.51±0.05 | 0.7±0.12 | 0.58±0.09 |
| 2.5% | 2-Oxide glass | 1.5±0.2 | 1.6±0.3 | 1.3±0.2 |
| | 3-Oxide glass | 1.2±0.2 | 1.5±0.2 | 1.3±0.2 |
| | 4-Oxide glass | 1.4±0.3 | 1.7±0.4 | 1.5±0.3 |
| 5.0% | 2-Oxide glass | 1.5±0.1 | 1.6±0.2 | 1.4±0.2 |
| | 3-Oxide glass | 1.3±0.2 | 1.5±0.2 | 1.2±0.2 |
| | 4-Oxide glass | 1.4±0.1 | 1.6±0.2 | 1.3±0.1 |

The values of the hydroxyapatite/glass materials are much higher than those for hydroxyapatite. A slight decrease in the fracture toughness was found for the material fired at 1350°C.

**Examples 4 to 6**

The following glasses were produced according to the method of Examples 1 to 3, the compositions being given in mole percent.

| | **Example 4** | **Example 5** | **Example 6** |
|---|---|---|---|
| | (C2P) | (C3P) | (C5P) |
| Ca0 | 20 | 30 | 50 |
| $P_20_5$ | 80 | 70 | 50 |

Each of the glasses was added to hydroxyapatite powder in amounts of 2.5% and 5.0% by weight according to the procedures as described in Examples 1 to 3.

Bending strength and Young's modulus measurements were obtained for each sample according to the procedures as described in Examples 1 to 3. The bending strengths for the hydroxyapatite compositions containing 2.5% and 5.0% by weight of the glasses of Examples 4 to 6 are shown in Figures 2 and 3, whilst the Young's moduli for the hydroxyapatite compositions containing 2.5% and 5.0% by weight of the glasses of Examples 4 to 6 are shown in Figures 4 and 5. In each figure results are also given for hydroxyapatite (HA) compositions not including any glass.

The compositions of Example 6 containing 2.5% and 5% by weight of the C5P glass fired at 1200°C were subjected to scanning electron microscopy using a JEOL field 6300F field emission electron microscope. The samples were gold coated and then examined at a working distance of 8mm with an accelerating voltage of 2-3 kv.

Figure 6 shows the grain boundaries for a sample containing 2.5 wt% C5P. The sample can be seen to be well sintered with little porosity present.

Figure 7 shows a sample containing 5.0 wt% C5P. The sample is well densified, but grain growth has occured compared with the sample reinforced with only 2.5 wt% C5P. Also the grains and grain boundaries show signs of etching, denoted by the pockmarked appearance of the surface.

## Claims

1. A sintered enhanced hydroxyapatite which comprises hydroxyapatite, $Ca_{1O}(P0_4)_6(OH)_2$, and a biocompatible glass based upon Ca0 and $P_20_5$.

2. A material as claimed in claim 1 wherein the biocompatible glass is contained in an amount of less than 10% by weight based on the weight of hydroxyapatite.

3. A material as claimed in claim 2 wherein the biocompatible glass is contained in an amount of from 2.5% to 5.0% by weight based on the weight of hydroxy-apatite.

4. A material as claimed in any one of the preceding claims wherein the biocompatible glass is a two oxide Ca0-$P_20_5$ glass.

5. A material as claimed in claim 4 wherein the glass comprises a mole ratio of Ca0:$P_20_5$ of 50:50.

6. A material as claimed in any one of claims 1 to 3 wherein the biocompatible glass is a three oxide Ca0-$P_20_5$-$Na_20$ glass.

7. A material as claimed in any one of claims 1 to 3 wherein the biocompatible glass is a four oxide Ca0-$P_20_5$-$Na_20$-$Al_20_3$ glass.

8. A method for the preparation of a material as claimed in claim 1 which comprises sintering a mixture of hydroxyapatite, $Ca_{10}(P0_4)_6(OH)_2$, and a biocompatible glass based upon Ca0 and $P_20_5$ at a temperature of above 1200°C.

9. A method as claimed in claim 8 wherein the sintering is carried out at a temperature in the range of from 1250° to 1350°C.

10. A method as claimed in claim 8 or claim 9 wherein the mixture of hydroxyapatite and biocompatible glass has a particle of less than 75 micrometres.

11. A method as claimed in any one of claims 8 to 10 wherein the biocompatible glass is contained in an amount of less than 10% by weight based on the weight of hydroxyapatite.

12. A method as claimed in claim 11 wherein the biocompatible glass is contained in an amount of from 2.5% to 5.0% by weight based on the weight of hydroxy-apatite.

13. A method as claimed in any one of claims 8 to 12 wherein the biocompatible glass is a two oxide $Ca0-P_20_5$ glass.

14. A method as claimed in claim 13 wherein the glass comprises a mole ratio of $Ca0:P_20_5$ of 50:50.

15. A method as claimed in any one of claims 8 to 12 wherein the biocompatible glass is a three oxide $Ca0-P_20_5-Na_20$ glass.

16. A method as claimed in any one of claims 8 to 12 wherein the biocompatible glass is a four oxide $Ca0-P_20_5-Na_20-Al_20_3$ glass.

17. A bone implant or bone filler which comprises an enhanced hydroxyapatite as claimed in any one of claims 1 to 7.

18. An artificial joint which comprises a coating of an enhanced hydroxyapatite as claimed in any one of claims 1 to 7 on at least a part of a metal or alloy joint.

19. A composition which comprises an enhanced hydroxyapatite as claimed in any one of claims 1 to 7 together with a pharmaceutically acceptable diluent or carrier.

20. A composition as claimed in claim 19 which is a bone cement or a dental cement.

FIG. 1.

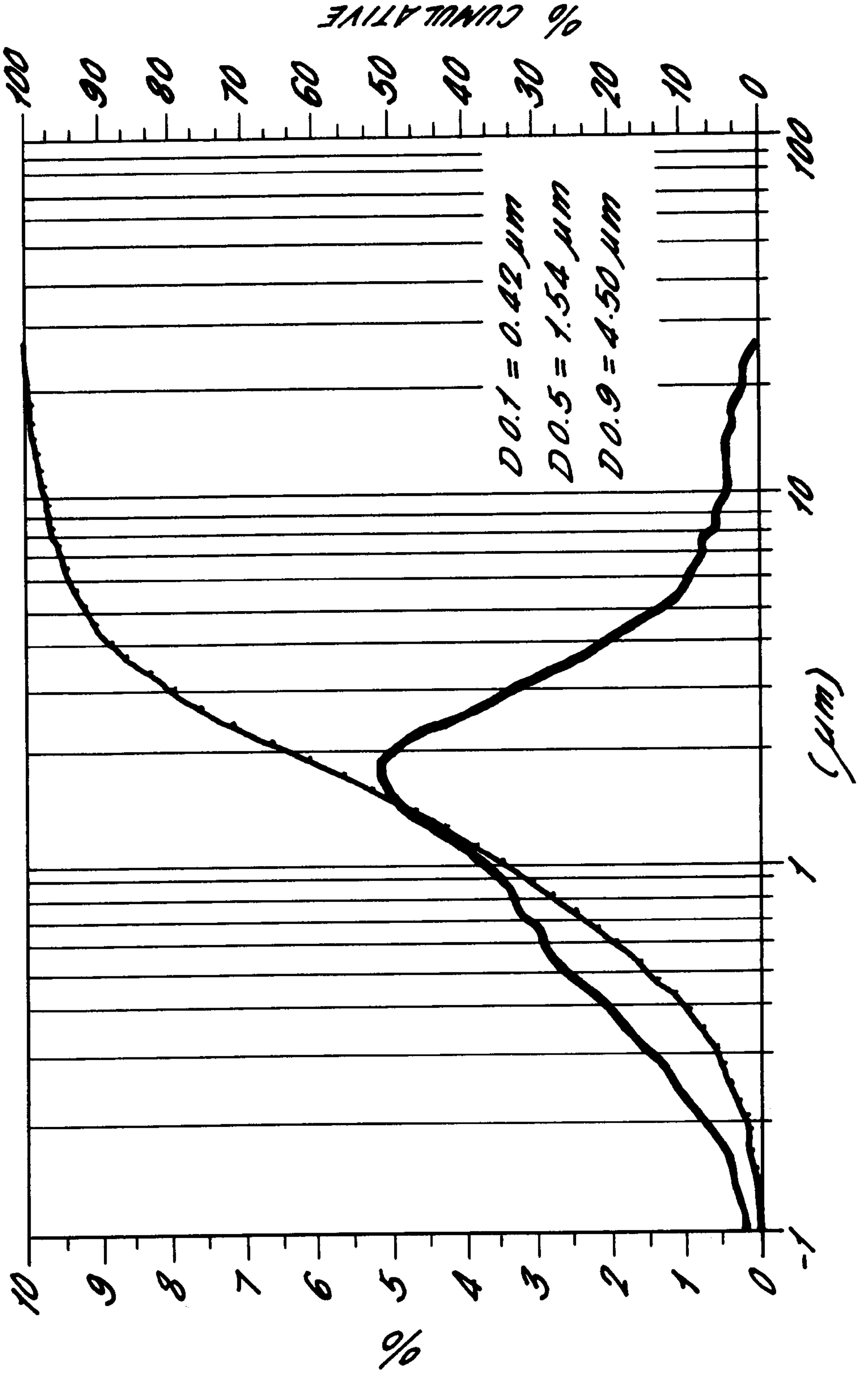


PARTICLE SIZE DISTRIBUTION PROFILE FOR HYDROXYAPATITE/
GLASS POWDER.

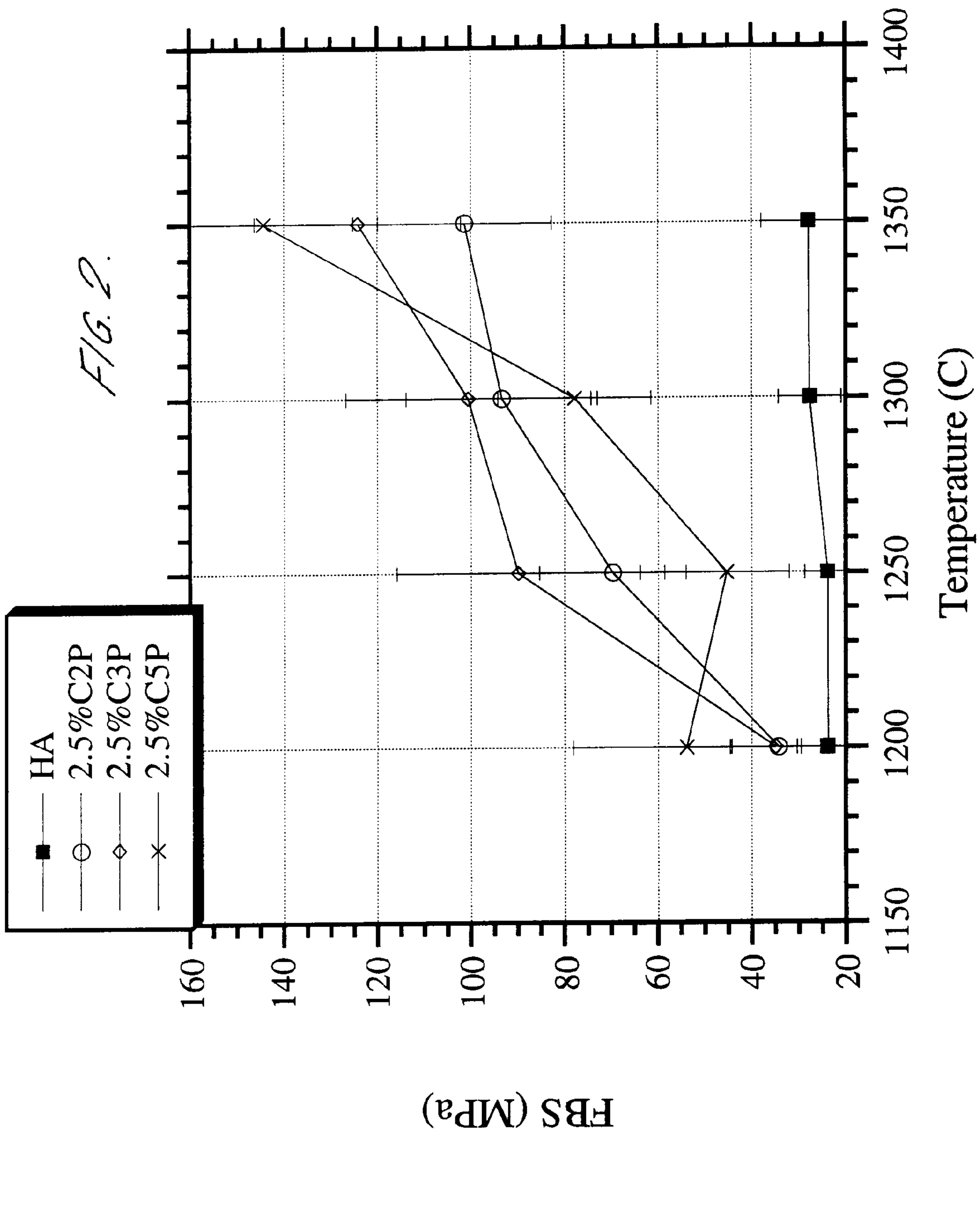
HA
2.5%C2P
2.5%C3P
2.5%C5P
FBS (MPa)
160
140
120
100
80
60
40
20
Temperature (C)
1150
1200
1250
1300
1350
1400

FIG. 2.

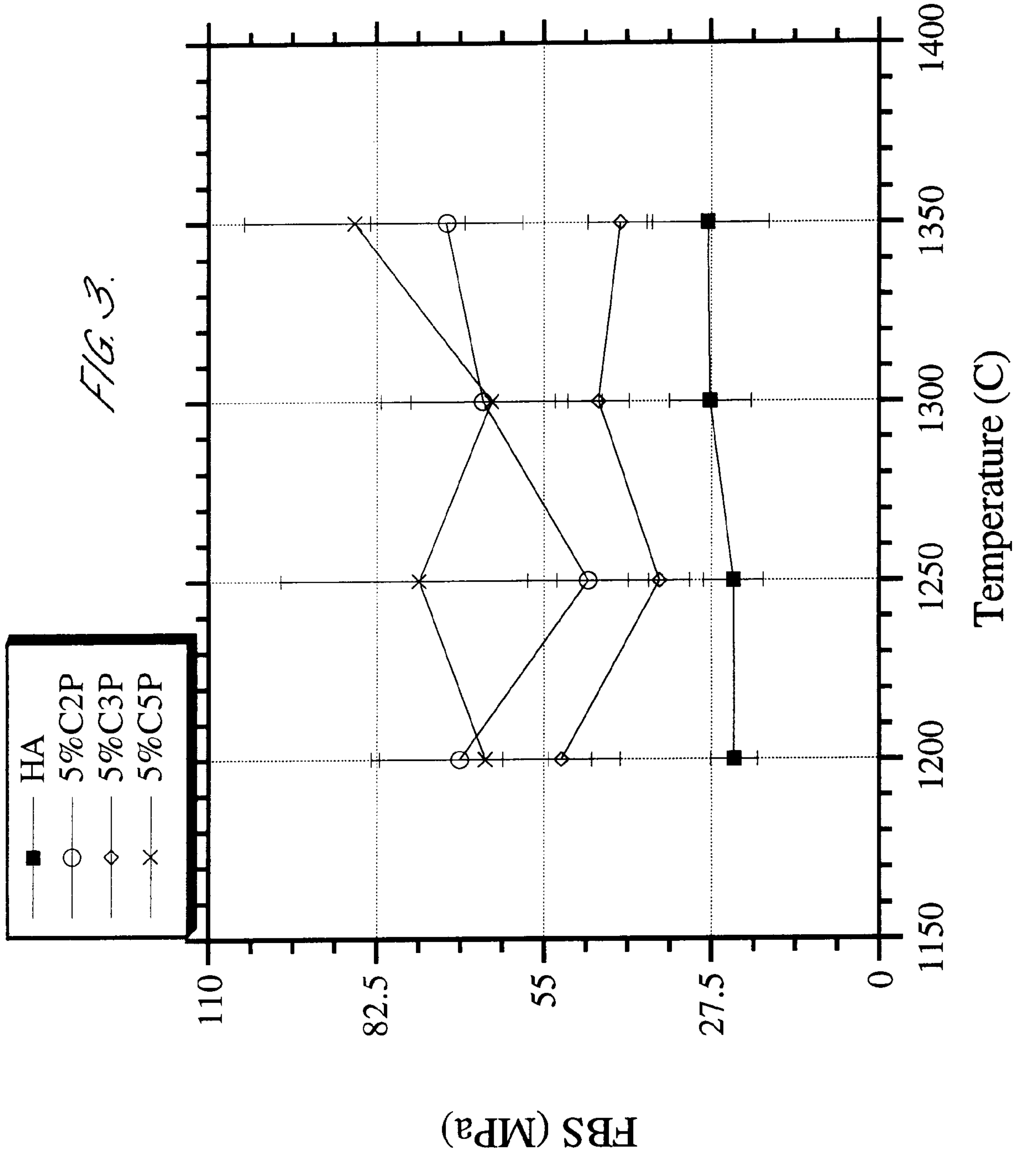
HA
5%C2P
5%C3P
5%C5P
110
82.5
55
27.5
0
FBS (MPa)
1150
1200
1250
1300
1350
1400
Temperature (C)

FIG. 3.

FIG. 4.

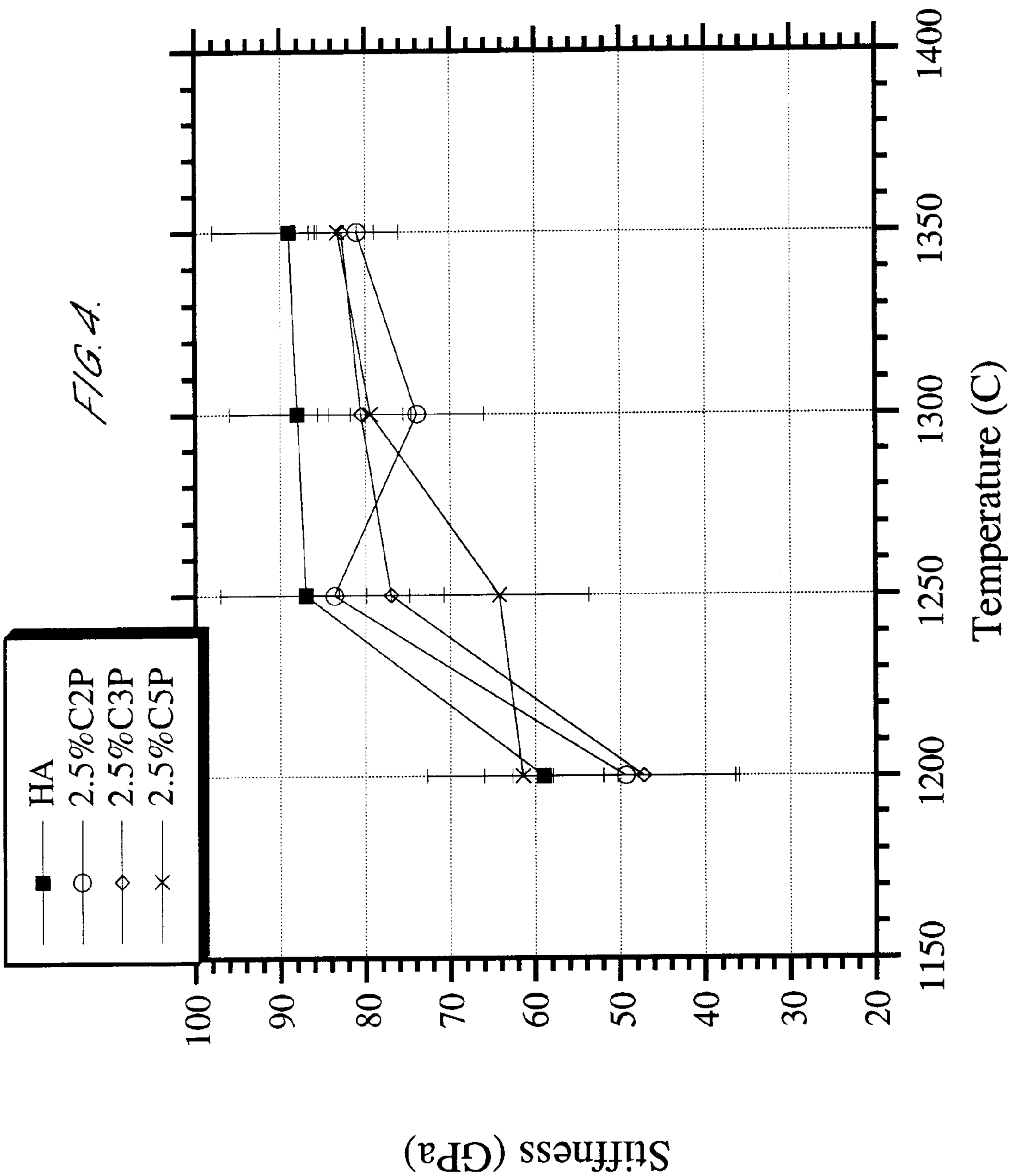
HA
2.5%C2P
2.5%C3P
2.5%C5P
Stiffness (GPa)
100
90
80
70
60
50
40
30
20
Temperature (C)
1150
1200
1250
1300
1350
1400

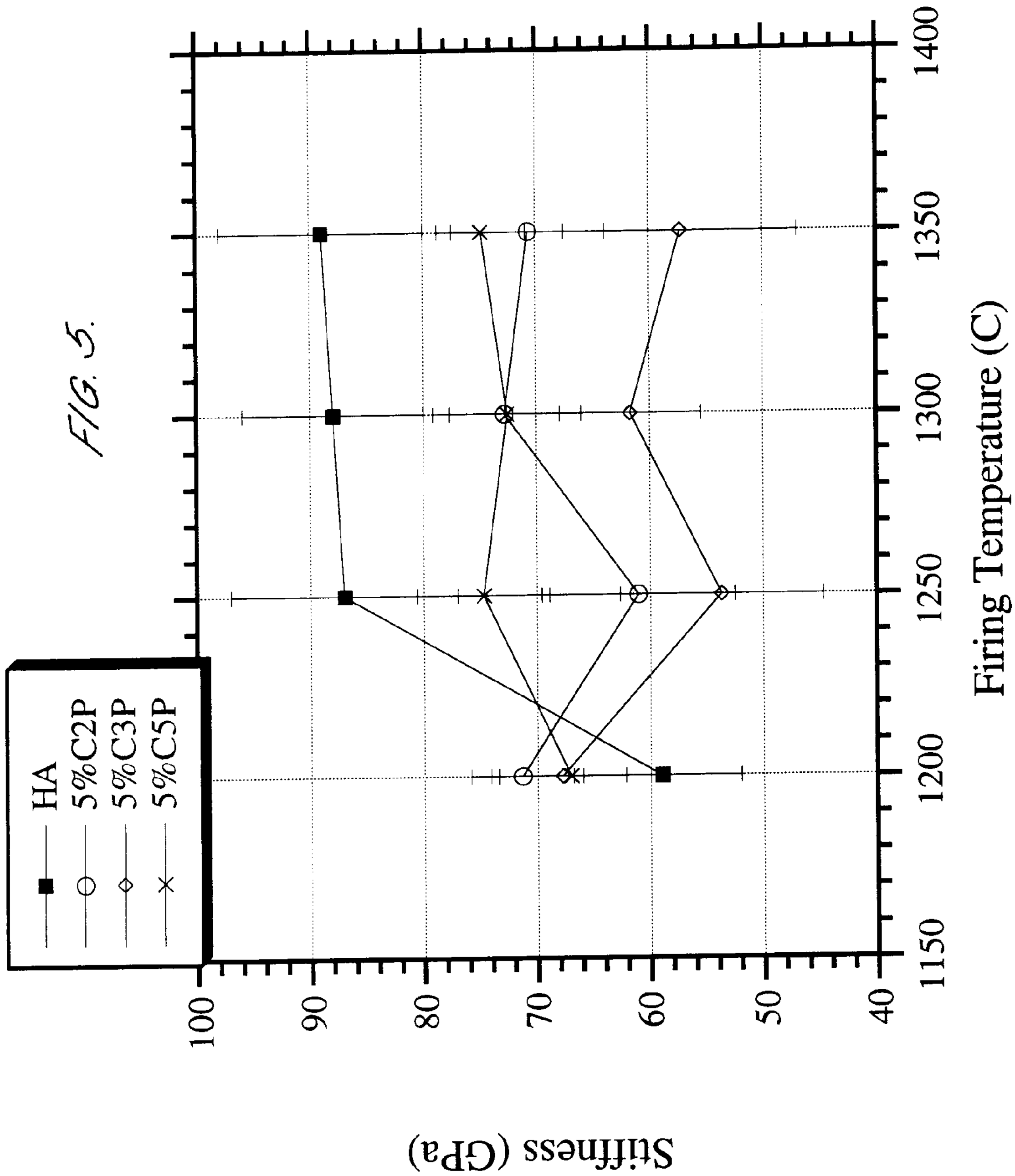
HA
5%C2P
5%C3P
5%C5P
Stiffness (GPa)
100
90
80
70
60
50
40
Firing Temperature (C)
1150
1200
1250
1300
1350
1400

FIG. 5.

FIG. 6.

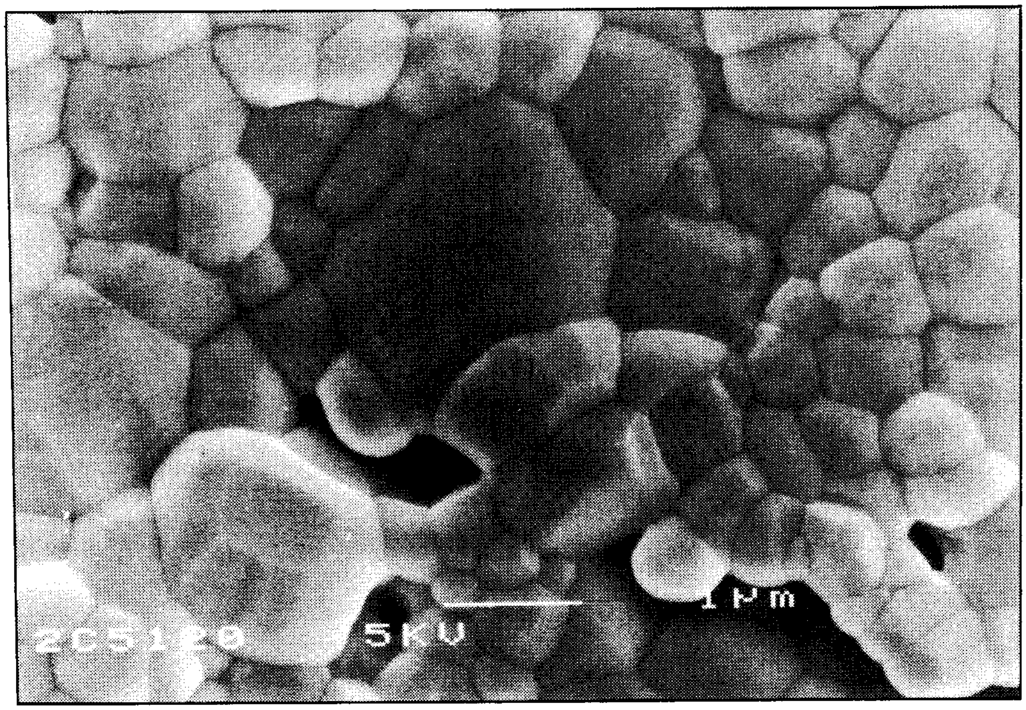


FIG. 7.

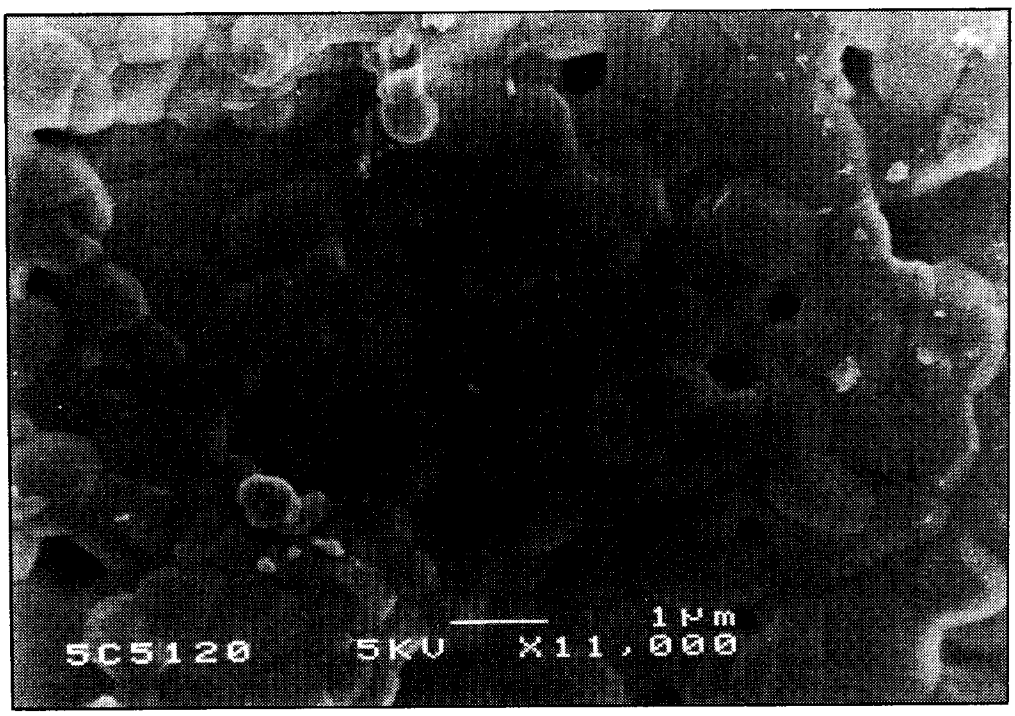

European Patent Office

# EUROPEAN SEARCH REPORT

Application Number

EP 93 30 4958

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | DE-A-2 652 611 (TOKYO MEDICAL AND DENTAL UNIVERSITY) * page 9, line 23 - line 28; claims 1-5; example 2; table 1 * --- | 1-20 | C04B35/00 A61L27/00 A61L25/00 A61K6/033 |
| A | US-A-4 708 652 (T. FUJIU ET AL.) * column 1, line 67 - column 2, line 24 * --- | 1-20 | |
| A | DATABASE WPI Week 8632, Derwent Publications Ltd., London, GB; AN 86-208809 & JP-A-61 141 660 (NIPPON KOGAKU KK) 28 June 1986 * abstract * --- | 1-20 | |
| A | GB-A-2 178 422 (C.F. DRAKE) * page 1, line 29 - line 34; claims 1-12; table 1 * ----- | 1,4,5-8, 13,15,16 | |

TECHNICAL FIELDS SEARCHED (Int. Cl.5 )

C04B
A61K
A61L
C03C

The present search report has been drawn up for all claims

| Place of search | Date of completion of the search | Examiner |
|---|---|---|
| THE HAGUE | 10 SEPTEMBER 1993 | LUETHE H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)